# FASCICULE DE BREVET EUROPEEN

(11) **EP 0 708 778 B1**
(45) Date de publication et mention de la délivrance du brevet: **05.02.1997**
(21) Numéro de dépôt: 94922903.3
(22) Date de dépôt: 11.07.1994
(51) Int. Cl.: C07D 491/14, C07D 487/14, C07D 495/14, A61K 31/495, A61K 31/415, A61K 31/34, A61K 31/38, A61K 31/40

(54) **DERIVES D'IMIDAZO [1,2-A]PYRAZINE-4-ONE/UTILES COMME ANTAGONISTES DES RECEPTEURS AMPA ET NMDA**
IMIDAZO [1,2-A] PYRASIN-4-ON-DERIVATE WELCHE ALS AMPA UND NMDA RECEPTOR ANTAGONISTEN DIENEN
IMIDAZO[1,2-A]PYRAZIN-4-ONE DERIVATIVES FOR USE AS AMPA AND NMDA RECEPTOR ANTAGONISTS

(30) Priorité: 16.07.1993 FR 9308753
(43) Date de publication de la demande: 01.05.1996
(73) Titulaire: RHONE-POULENC RORER S.A., 92160 Antony (FR)
(72) Inventeur: ALOUP, Jean-Claude, F-94290 Villeneuve-le-Roi (FR); MIGNANI, Serge, F-92290 Chatenay-Malabry (FR)
(74) Mandataire: Savina, Jacques
(86) Numéro de dépôt international: FR9400865
(87) Numéro de publication internationale: WO9502602

(56) Documents cités:
- WO-A-91/16325
- JOURNAL OF MEDICINAL CHEMISTRY, vol.35, no.18, 1992, WASHINGTON US pages 3319 - 3324 L.A. MCQUAID ET AL. 'Synthesis and Excitatory Amino Acid Pharmacology of a Series of Heterocyclic-Fused Quinoxalinones and Quinazolinones'

## Description

Des dérivés de 1,2,4-triazolo[4,3-a]quinoxalin-4(5H)one, tétrazolo[1,5-a]quinoxalin-4(5H)one, pyrazolo[1,5-c]quinazolin-5(6H)one et imidazo[1,2-a]quinoxalin-4(5H)one ont été testés comme antagonistes du récepteur AMPA et du site glycine du récepteur NMDA (J. Med. Chem., 35, 3319-3324 (1992)). Par ailleurs, des dérivés de benzothiéno[2,3-b]pyrazine-2,3(1H,4H)dione sont décrits dans la demande de brevet WO91/16325 comme antagonistes du site glycine du récepteur NMDA.

La présente invention concerne les composés de formule : 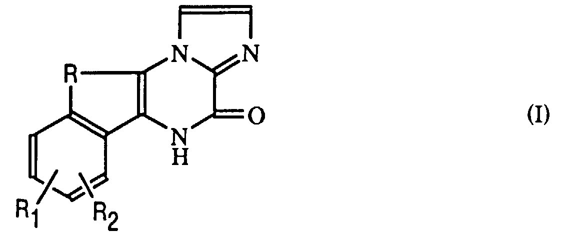 leurs sels, leur préparation et les médicaments les contenant.

Dans la formule (I),
- R représente un atome d'oxygène ou de soufre ou un radical NH ou N-alk,
- R₁ et R₂, identiques ou différents, représentent des atomes d'hydrogène ou d'halogène ou des radicaux alkyle, alcoxy, amino, acylamino, -NH-CO-NH-Ar, -N=CH-N(alk)alk', nitro, cyano, phényle, imidazolyle ou SO₃H,
- Ar représente un radical phényle,
- alk représente un radical alkyle,
- alk' représente un radical alkyle identique ou différent de alk.

Sauf mention contraire, dans les définitions qui précédent et celles qui suivent, les radicaux alkyle et alcoxy contiennent 1 à 4 atomes de carbone en chaîne droite ou ramifiée, les radicaux et portions acyle contiennent 2 à 5 atomes de carbone et les atomes d'halogène sont choisis parmi le fluor, le chlore, le brome et l'iode.

Les composés de formule (I) pour lesquels R₁ et/ou R₂ représentent un radical -N=CH-N(alk)alk' présentent des formes isomères (E et Z). Ces isomères et leurs mélanges font partie de l'invention.

Les composés de formule (I) pour lesquels R représente un atome d'oxygène peuvent être préparés par désalkylation, déshydratation et désalification des dérivés de formule : 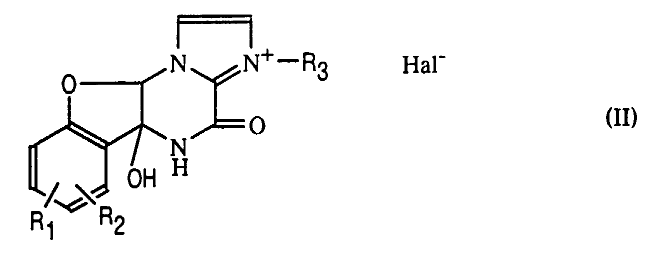 dans laquelle R₁ et R₂ ont les mêmes significations que dans la formule (I), R₃ représente un radical alkyle et Hal représente un atome d'halogène. De préférence, Hal représente un atome de brome.

Cette réaction s'effectue, de préférence, en présence d'imidazole, par chauffage à une température comprise entre 100 et 200°C.

Les dérivés de formule (Il) peuvent être obtenus par action d'un 1-alkyl 1H-imidazole-2-carboxamide sur un dérivé de formule : 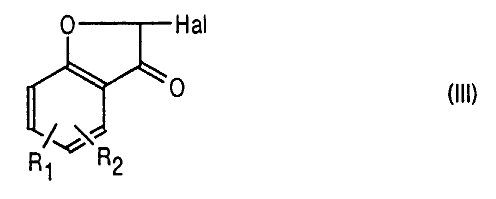 dans laquelle R₁ et R₂ ont les mêmes significations que dans la formule (I) et Hal représente un atome d'halogène. De préférence, Hal représente un atome de brome.

Cette réaction s'effectue généralement au sein d'un solvant inerte tel que l'acétonitrile, à la température d'ébullition du milieu réactionnel.

Les 1-alkyl 1H-imidazole-2-carboxamide peuvent être obtenus par application ou adaptation de la méthode décrite par D. D. DAVEY, J. Org. Chem., 52, 4379 (1987).

Les dérivés de formule (III) peuvent être obtenus par halogénation des 3-coumaranones correspondantes, au moyen d'un agent d'halogénation, au sein d'un solvant inerte tel qu'un solvant chloré (chlorure de méthylène, chloroforme par exemple), à une température voisine de -15°C. De préférence, on utilise le brome ou le chlore.

Les 3-coumaranones sont commercialisées ou peuvent être obtenues par application ou adaptation de la méthode décrite par A. R. DESHPANDE et coll., Synth. Commun., 20 (6), 809 (1990) et G. SCHENK et coll., Tetrahedron Lett., (19), 2375 (1968).

Les composés de formule (I) pour lesquels R représente un atome de soufre peuvent être préparés par cyclisation d'un dérivé de formule : 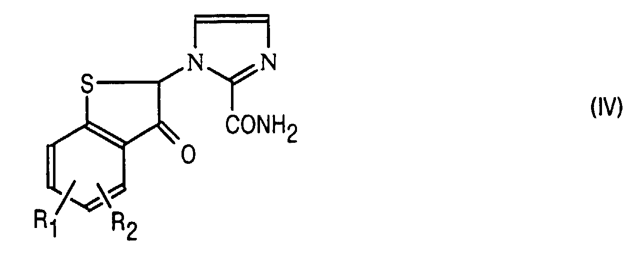 dans laquelle R₁ et R₂ ont les mêmes significations que dans la formule (I).

Cette cyclisation s'effectue généralement au moyen d'un acide tel que l'acide chlorhydrique en solution aqueuse, à une température voisine de 20°C.

Les dérivés de formule (IV) peuvent être obtenus par action d'ammoniac sur un dérivé de formule : 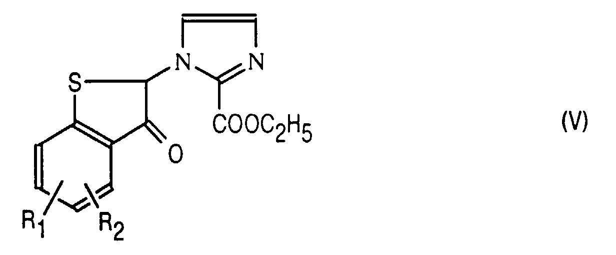 dans laquelle R₁ et R₂ ont les mêmes significations que dans la formule (I).

Cette réaction s'effectue généralement au sein d'un solvant inerte tel qu'un alcool, à une température comprise entre 20°C et la température d'ébullition du milieu réactionnel.

Les dérivés de formule (V) peuvent être obtenus par condensation d'imidazole-2-carboxylate d'éthyle sur un dérivé de formule : 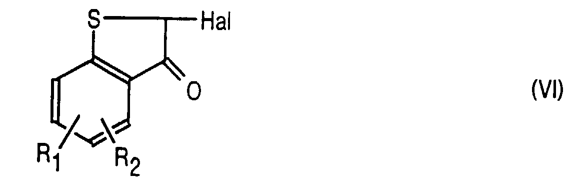 dans laquelle R₁ et R₂ ont les mêmes significations que dans la formule (I) et Hal représente un atome d'halogène et, de préférence, un atome de brome.

Cette réaction s'effectue, au sein d'un solvant inerte tel qu'un alcool (méthanol, éthanol par exemple), à la température d'ébullition du milieu réactionnel.

L'imidazole-2-carboxylate d'éthyle peut être obtenu selon la méthode décrite dans le brevet US 3600399.

Les dérivés de formule (VI) peuvent être obtenus par application ou adaptation de la méthode décrite par Z.I. MIROSHNICHENKO et M.A. AL'PEROVICH, J. Gen. Chem. USSR, 32, 1218 (1962).

Les composés de formule (I) pour lesquels R représente un radical NH peuvent être préparés par hydrolyse d'un dérivé de formule : 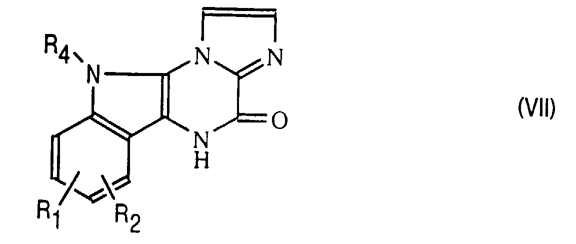 dans laquelle R₁ et R₂ ont les mêmes significations que dans la formule (I) et R₄ représente un radical acyle (2-5C).

Cette réaction s'effectue au sein d'un solvant inerte tel qu'un amide (diméthylformamide par exemple), l'eau ou un mélange de ces solvants, à une température variant de 5°C à la température d'ébullition du milieu réactionnel.

Les dérivés de formule (VII) pour lesquels R₄ représente un radical acyle (2-5C) peuvent être obtenus par cyclisation des dérivés de formule : 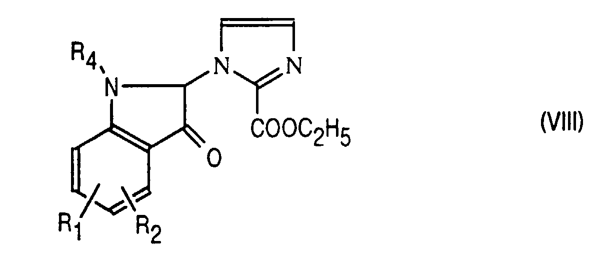 dans laquelle R₁ et R₂ ont les mêmes significations que dans la formule (I) et R₄ représente un radical acyle (2-5C).

Cette cyclisation s'effectue, de préférence, au sein d'un solvant inerte tel que l'acide acétique, en présence d'acétate d'ammonium, à la température d'ébullition du milieu réactionnel.

Les dérivés de formule (VIII) peuvent être obtenus par chauffage à une température voisine de 120°C d'imidazole-2-carboxylate d'éthyle et d'un dérivé de formule : 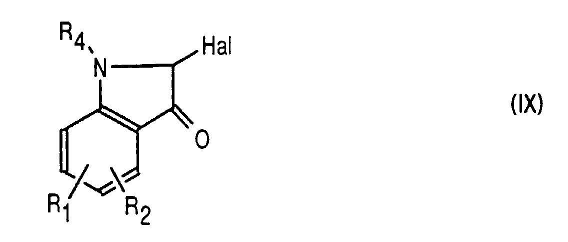 dans laquelle R₁ et R₂ ont les mêmes significations que dans la formule (I), R₄ représente un radical acyle (2-5C) et Hal représente un atome d'halogène et, de préférence, de brome.

Les dérivés de formule (IX) peuvent être obtenues par application ou adaptation de la méthode décrite par V. S. VELEZHEVA et coll., Khim. Farm. Zh., 24 (12), 46 (1990).

Les composés de formule (I) pour lesquels R représente un radical N-alk peuvent être préparés par alkylation d'un composé de formule (I) correspondant pour lequel R représente un radical NH.

Cette réaction s'effectue, de préférence, au moyen d'un halogénure d'alkyle, en présence d'une base organique telle que la triéthyamine, une base inorganique telle qu'un hydroxyde de métal alcalin (soude, potasse par exemple), un carbonate de métal alcalin (carbonate de sodium par exemple), éventuellement en présence de bromure de tétrabutylammonium, au sein d'un solvant inerte tel que le diméthylsulfoxyde, le diméthylformamide ou la pyridine, à une température comprise entre 20 et 50°C.

Les composés de formule (I) peuvent être purifiés par les méthodes connues habituelles, par exemple par cristallisation, chromatographie ou extraction.

Les isomères E et Z des composés de formule (I) pour lesquels R₁ et/ou R₂ représentent un radical -N=CH-N(alk)alk' peuvent être séparés par les méthodes connues habituelles, par exemple par cristallisation ou chromatographie.

Les composés de formule (I) comportant un reste basique peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un reste acide peuvent éventuellement être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'une amine sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les composés de formule (I) présentent des propriétés pharmacologiques intéressantes. Ces composés sont des antagonistes du récepteur de l'acide α-amino-3-hydroxy-5-méthyl-4-isoxazolepropionique (AMPA), connu aussi sous le nom de récepteur du quisqualate.

Par ailleurs, les composés de formule (I) sont des antagonistes non compétitifs du récepteur N-méthyl-D-aspartate (NMDA) et, plus particulièrement, ce sont des ligands pour les sites modulateurs de la glycine du récepteur NMDA.

Ces composés sont donc utiles pour traiter ou prévenir toutes les ischémies (telles l'ischémie focale ou globale) consécutives à des accidents vasculaires cérébraux, un arrêt cardiaque, une hypotension artérielle, une intervention chirurgicale cardiaque ou pulmonaire ou une hypoglycémie sévère. Ils sont également utiles dans le traitement des effets dus à une anoxie, qu'elle soit périnatale ou consécutive à une noyade ou à des lésions cérébro-spinales. Ces composés peuvent également être utilisés pour traiter ou prévenir l'évolution de maladies neurodégénératives, de la chorée d'HUNTINGTON, de la maladie d'ALZHEIMER, de la sclérose latérale amyotrophique, de l'atrophie olivo-pontocérébelleuse et de la maladie de PARKINSON. Ces composés peuvent aussi être utilisés vis-à-vis des manifestations épileptogènes et/ou convulsives, pour le traitement des traumatismes cérébraux ou spinaux, des traumatismes liés à la dégénérescence de l'oreille interne (R. PUJOL et coll., Neuroreport, 3, 299-302 (1992) ou de la rétine (J.L. MONSINGER et coll., Exp. Neurol., 113, 10-17 (1991), de l'anxiété (KEHNE et coll., Eur. J. Pharmacol., 193, 283 (1991)), de la dépression (TRULLAS et coll.,Eur. J. Pharmacol., 185, 1 (1990)), de la schizophrénie (REYNOLDS, TIPS, 13, 116 (1992)), du syndrome de TOURETTE, des encéphalopathies hépatiques, en tant qu'analgésiques (DICKENSON et coll., Neurosc. Letters, 121, 263 (1991)), antiinflammatoires (SLUTA et coll., Neurosci. Letters, 149, 99-102 (1993)) antianorexiques (SORRELS et coll., Brain Res., 572, 265 (1992)), antimigraineux, antiémétiques et pour traiter les empoisonnements par des neurotoxines ou d'autres substances agonistes du récepteur NMDA, ainsi que les troubles neurologiques associés aux maladies virales telles que le sida (LIPTON et coll., Neuron, 7, 111 (1991)), la rage, la rougeole et le tétanos (BAGETTA et coll., Br. J. Pharmacol., 101, 776 (1990)). Ces composés sont aussi utiles pour la prévention des symptômes d'abstinence aux drogues et à l'alcool et de l'inhibition de l'accoutumance et de la dépendance aux opiacés. Ils peuvent également être utilisés dans le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie de LEAD et la déficience en sulfite oxydase.

L'affinité des composés de formule (I) vis-à-vis du récepteur AMPA a été déterminée en étudiant l'antagonisme de la fixation spécifique du [³H]-AMPA sur des membranes de cortex cérébral de rat (HONORE et coll., Neuroscience letters, 54, 27 (1985)). Le [³H]-AMPA est mis à incuber en présence de 0,2 mg de protéines à 4°C pendant 30 minutes dans du tampon KH₂PO₄ 10mM, KSCN 100mM, pH7,5. La fixation non spécifique est déterminée en présence de L-glutamate 1mM. La radioactivité liée est séparée par filtration sur filtres PHARMACIA (Printed Filtermate A). L'activité inhibitrice de ces produits est égale ou inférieure à 100 µM.

L'affinité des composés de formule (i) pour le site glycine lié au récepteur NMDA a été déterminée en étudiant l'antagonisme de la fixation spécifique du [³H]-DCKA sur des membranes de cortex cérébral de rat selon la méthode décrite par T. CANTON et coll., J. Pharm. Pharmacol., 44, 812 (1992). Le [³H]-DCKA (20nM) est mis à incuber en présence de 0,1 mg de protéines à 4°C pendant 30 minutes dans du tampon HEPES 50 mM, pH7,5. La fixation non spécifique est déterminée en présence de glycine 1mM. La radioactivité liée est séparée par filtration sur filtres Whatman GF/B. L'activité inhibitrice de ces produits est égale ou inférieure à 100 µM.

Les composés de formule (I) présentent une toxicité faible. Leur DL50 est supérieure à 50 mg/kg par voie IP chez la souris.

Sont particulièrement intéressants les composés de formule (I) pour lesquels R₁ et R₂ représentent des atomes d'hydrogène et R représente un atome d'oxygène, de soufre ou un radical NH ou N-alk.

### EXEMPLE 1

Une solution de 6 g de bromure de 5a-hydroxy-3-méthyl-4-oxo-5a,10a-dihydro-5H-benzofuro[3,2-e]imidazo[1,2-a]pyrazinium dans 30 g d'imidazole fondu est chauffée pendant 4 heures à 155°C, conservée après refroidissement pendant 15 heures à une température voisine de 20°C puis de nouveau chauffée pendant 6 heures à 165°C et pendant 2 heures à 175°C. Le mélange est refroidi à une température voisine de 100°C et, après addition lente de 50 ml d'eau distillée, versé sur 250 ml d'eau distillée, refroidi à 20°C et filtré. Le filtrat est concentré au seizième de son volume sous pression réduite (15 mm Hg; 2 kPa) à 60°C et refroidi à 20°C. Le solide est séparé par filtration, séché sous pression réduite (15 mm Hg; 2 kPa) à 20°C. Le produit obtenu (1,4 g) est dissous dans 120 ml d'acide acétique bouillant et la solution, additionnée de 0,1 g de noir décolorant, est filtrée à chaud et refroidie à 20°C. Les cristaux sont séparés par filtration, lavés 2 fois avec 100 ml au total d'éthanol et avec 50 ml d'éther éthylique puis séchés sous pression réduite (1 mm Hg; 0,13 kPa) à 80°C. On obtient ainsi 0,8 g de 5H-benzofuro[3,2-e]imidazo[1,2-a]pyrazine-4-one se sublimant à partir de 320°C [Spectre de R.M. N.: (300 MHz; DMSO d6; δ en ppm) : 7,45 (ab limite, 2 H : -H7 et -H8); 7,68 et 8,27 (2s, 1H chacun : -H de l'imidazole); 7,78 et 7,92 (2mt, 1H chacun : -H6 et -H9); 12,42 (mf, 1 H : -NH-)].

Le bromure de 5a-hydroxy-3-méthyl-4-oxo-5a,10a-dihydro-5H-benzofuro [3,2-e]imidazo[1,2-a]pyrazinium peut être préparé de la façon suivante : une solution de 6 g de 2-bromo-2H-benzofuranne-3-one dans 20 ml d'acétonitrile est ajoutée à une solution bouillante de 1-méthyl-1H-imidazole-2-carboxamide dans 30 ml d'acétonitrile. Le mélange est agité pendant 8 heures à l'ébullition et, après refroidissement à 20°C, les cristaux sont séparés par filtration, lavés 2 fois avec 50 ml au total d'acétone et 50 ml d'éther éthylique puis séchés sous pression réduite (15 mm Hg; 2 kPa) à 20°C. On obtient ainsi 6,7 g de bromure de 5a-hydroxy-3-méthyl-4-oxo-5a,10a-dihydro-5H-benzofuro[3,2-e]imidazo[1,2-a]pyrazinium [Spectre de R.M. N. : (200 MHz; DMSO d6; δ en ppm) : 4,15 (s, 3H : N⁺-CH3); 6,74 (s, 1H : CH); 7,08 et 7,60 (2d, J=7,5 Hz, 1H chacun : -H6 et -H9), 7,18 et 7,45 (2t, J=7,5 Hz, 2H : -H7 et -H8); 8,15 et 8,25 (2d, J=1 Hz, 1H chacun : -H de l'imidazole).

La 2-bromo-2H-benzofuranne-3-one peut être préparée de la façon suivante : 18,4 g de brome sont ajoutés à une solution maintenue à -10°C de 31,7 g de 2H-benzofuranne-3-one dans 460 ml de chlorure de méthylène. Après 30 minutes d'agitation, 18,4 g de brome sont de nouveau ajoutés et le mélange est agité pendant 2 heures 30 minutes à la même température, neutralisé par 100 ml d'une solution aqueuse saturée d'hydrogénocarbonate de sodium, lavé avec 100 ml d'une solution aqueuse saturée de chlorure de sodium, séché par du sulfate de magnésium anhydre et concentré à sec sous pression réduite (15 mm Hg; 2 kPa) à 45°C. On obtient ainsi 46,8 g de 2-bromo-2H-benzofuranne-3-one fondant à 60°C.

Le 1-méhyl-1H-imidazole-2-carboxamide peut être préparé selon le procédé décrit par D. D. DAVEY, J. Org. Chem., 52, 4379 (1987).

### EXEMPLE 2

1 g de de 1-(3-oxo-2,3-dihydro-benzo[b]thiophène-2-yl)-imidazole-2-carboxamide est dissous dans 50 ml d'une solution aqueuse 10N d'acide chlorhydrique et après 10 minutes d'agitation à 20°C, les cristaux sont séparés par filtration, lavés 2 fois avec 10 ml au total d'eau distillée et avec 5 ml d'acétone puis séchés sous pression réduite (1 mm Hg; 0,15 kPa) à 100°C. On obtient ainsi 0,61 g de chlorhydrate de 5H-[1]benzothiéno[3,2-e] imidazo[1,2-a]pyrazine-4-one (0,4 mole d'acide par mole de base) se décomposant sans fondre vers 320°C [Spectre de R.M.N.: (300 MHz; DMSO d6; δ en ppm) : 7,52 et 7,58 (2dt, J=7,5 et 1 Hz, 1 H chacun : -H7 et -H8); 7,78 et 8,35 (2s larges, 1H chacun : -H de l'imidazole); 8,15 et 8,36 (2d larges, 1H chacun : -H6 et -H9); 12,92 (mf, 1H : -NH-)].

Le 1-(3-oxo-2,3-dihydro-benzo[b]thiophène-2-yl)-imidazole-2-carboxamide peut être préparé de la façon suivante : une solution de 1,92 g de 1-(3-oxo-2,3-dihydro-benzo[b]thiophène-2-yl)-imidazole-2-carboxylate d'éthyle dans 130 ml d'une solution 5N de méthanol ammoniacal est agitée pendant 15 heures à une température voisine de 20°C puis concentrée à sec sous pression réduite (15 mm Hg; 2 kPa) à 40°C. Le produit obtenu (1,66 g) est mis en suspension dans 30 ml d'oxyde d'isopropyle, filtré, lavé 2 fois avec 10ml au total d'oxyde d'isopropyle et séché sous pression réduite (15 mm Hg; 2 kPa) à 20°C. On obtient ainsi 1,5 g de 1-(3-oxo-2,3-dihydrobenzo[b]thiophène-2-yl)-imidazole-2-carboxamide fondant à 180°C.

Le 1-(3-oxo-2,3-dihydro-benzo[b]thiophène-2-yl)-imidazole-2-carboxylate d'éthyle peut être préparé de la façon suivante : 18 g de 2-bromo-2H-benzo[b]thiophène-3-one sont ajoutés à 50°C à une solution de 20,1 g d'imidazole-2-carboxylate d'éthyle dans 500 ml d'éthanol. Le mélange est chauffé à l'ébullition pendant 10 heures, refroidi à 20°C, filtré et le filtrat est concentré à sec sous pression réduite (15 mm Hg; 2 kPa) à 50°C. Le produit obtenu est chromatographié sur 2,7 kg de gel de silice neutre (0,020-0,045 mm) contenus dans une colonne de 9 cm de diamètre en éluant sous pression par un mélange chlorure de méthylène-acétate d'éthyle (80-20 en volumes) et en recueillant des fractions de 1 litre. Les fractions contenant le produit attendu sont concentrées à sec sous pression réduite (15 mm Hg; 2 kPa) à 50°C et le produit obtenu (3,4 g) est mis en suspension dans 50 ml d'éther de pétrole, filtré, lavé avec 10 ml d'éther de pétrole et séché à l'air. On obtient ainsi 3,2 g de 1-(3-oxo-2,3-dihydro-benzo[b]thiophène-2-yl)-imidazole-2-carboxylate d'éthyle fondant à 174°C.

La 2-bromo-2H-benzo[b]thiophène-3-one peut être préparée comme décrit par Z.I. MIROSHNICHENKO et M.A. AL'PEROVICH, J. Gen. Chem. USSR, 32, 1218 (1962).

### EXEMPLE 3

0,45 g de 5H,10H-imidazo[1,2-a]indolo[3,2-e]pyrazine-4-one est dissous à 60°C dans 50 ml de diméthylsulfoxyde et la solution est refroidie à 20°C. On ajoute ensuite 0,010 g de bromure de tétrabutylammonium, 0,8 g de soude et 0,3 g d'iodure d'éthyle. Le mélange est agité pendant 60 heures à une température voisine de 20°C, versé sur 400 ml d'eau distillée et acidifié avec 15 ml d'acide acétique. L'insoluble est séparé par filtration, lavé avec de l'eau distillée jusqu'à neutralité et séché sous pression réduite (5 mm Hg; 0,75 kPa) à 60°C. Le produit obtenu (0,3 m) est chromatographié sur 20 g de gel de silice neutre (0,020-0,045 mm) contenus dans une colonne de 2 cm de diamètre en éluant sous pression par un mélange chlorure de méthylène-méthanol (90-10 en volumes). Les fractions contenant le produit le plus polaire sont concentrées à sec sous pression réduite (15 mm Hg; 2 kPa) à 50°C. Le produit obtenu (120 mg) est mis en suspension 2 fois dans 20 ml au total de méthanol, filtré et séché sous pression réduite (1 mm Hg; 0,13 kPa) à 60°C. On obtient ainsi 0,03 g de 10-éthyl-5H,10H-imidazo[1,2-a]indolo[3,2-e] pyrazine-4-one fondant à 315°C.

### EXEMPLE 4

2 g de 10-acétyl-5H,10H-imidazo[1,2-a]indolo[3,2-e]pyrazine-4-one sont dissous dans 200 ml de diméthylformamide bouillant et la solution, additionnée de noir décolorant, est filtrée à chaud, diluée avec 50 ml d'eau distillée, refroidie et conservée pendant 16 heures à 5°C. Les cristaux sont séparés par filtration, lavés 3 fois avec 60 ml au total d'eau distillée et 3 fois avec 60 ml au total d'acétone puis séchés sous pression réduite (1 mm Hg; 0,15 kPa) à 100°C. On obtient ainsi 1,3 g de 5H,10H-imidazo[1,2-a] indolo[3,2-e]pyrazine-4-one se décomposant sans fondre au-dessus de 300°C [Spectre de R.M.N.: (200 MHz; DMSO d6; δ en ppm) : 7,20 et 7,30 (2t, J=7,5 Hz, 2H : -H7 et -H8); 7,67 et 8,12 (2s larges, 1H chacun : -H de l'imidazole); 7,58 et 7,93 (2d, J=7,5 Hz, 1H chacun : -H6 et -H9), 12,00 à 12,70 (mf, 2H : -NH)].

Le 10-acétyl-5H,10H-imidazo[1,2-a]indolo[3,2-e]pyrazine-4-one peut être préparé de la façon suivante : une solution de 8,4 g de 1-[2-(1-acétyl-3-oxo-indolinyl)]-imidazole-2-carboxylate d'éthyle et de 206 g d'acétate d'ammonium dans 300 ml d'acide acétique est chauffée à l'ébullition pendant 1 heure, refroidie à une température voisine de 20°C et additionnée de 50 ml d'eau distillée. Les cristaux sont séparés par filtration, lavés 2 fois avec 100 ml au total d'eau distillée et par 20 ml d'acétone puis séchés sous pression réduite (5 mm Hg; 0,65 kPa) à 60°C. On obtient ainsi 5,6 g de 10-acétyl-5H,10H-imidazo[1,2-a]indolo[3,2-e] pyrazine-4-one fondant à 340°C.

Le 1-[2-(1-acétyl-3-oxo-indolinyl)]-imidazole-2-carboxylate d'éthyle peut être préparé de la façon suivante : le mélange fondu de 10,2 g de 1-acétyl-2-bromoindoline-3-one et de 11,2 g d'imidazole-2-carboxylate d'éthyle est chauffé pendant 15 minutes à 120°C, et après refroidissement à 20°C, chromatographié sur 1 kg de gel de silice neutre (0,040-0,063 mm) contenus dans une colonne de 10 cm de diamètre en éluant sous pression par un mélange dichlorométhane-acétated'éthyle (60-40 en volumes) en recueillant des fractions de 500 ml. Les fractions 11 à 16 sont réunies et concentrées à sec sous pression réduite (5 mm Hg; 0,65 kPa) à 50°C. Le produit obtenu (9 g) est mis en suspension dans 100 ml d'éther de pétrole, filtré, lavé avec 20 ml d'éther de pétrole et séché sous pression réduite (5 mm Hg; 0,65 kPa) à 60°C. On obtient ainsi 8,6 g de 1-[2-(1-acétyl-3-oxo-indolinyl)]-imidazole-2-carboxylate d'éthyle fondant à 210°C.

La 1-acétyl-2-bromoindoline-3-one peut être préparée comme décrit par V.S. VELEZHEVA et coll., Khim. Farm. Zh., 24(12), 46 (1990).

Les médicaments selon l'invention sont constitués par un composé de formule (I) ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention des conditions qui requièrent l'administration d'un antagoniste du récepteur AMPA ou d'un antagoniste du récepteur NMDA. Ces composés sont notamment utiles pour traiter ou prévenir toutes les ischémies et en particulier l'ischémie cérébrale, les effets dus à une anoxie, l'évolution de maladies neurodégénératives, de la chorée d'HUNTINGTON, de la maladie d'ALZHEIMER, de la sclérose latérale amyotrophique, de l'atrophie olivo-pontocérébelleuse et de la maladie de PARKINSON, vis-à-vis des manifestations épileptogènes et/ou convulsives, pour le traitement des traumatismes cérébraux et spinaux, des traumatismes liés à le dégénérescence de l'oreille interne ou de la rétine, de l'anxiété, de la dépression, de la schizophrénie, du syndrome de TOURETTE, de l'encéphalopathie hépatique, en tant qu'analgésiques, antiinflammatoires, antianorexiques, antimigraineux, antiémétiques et pour traiter les empoisonnements par des neurotoxines ou d'autres substances agonistes du récepteur NMDA, ainsi que les troubles neurologiques associés aux maladies virales telles que le sida, la rage, la rougeole et le tétanos. Ces composés sont aussi utiles pour la prévention des symptomes d'abstinence aux drogues et à l'alcool et de l'inhibition de l'accoutumance et de la dépendance aux opiacés ainsi que pour le traitement des déficits liés à des anomalies mitochondriales telles que la myopathie mitochondriale, le syndrome de LEBER, l'encéphalopathie de WERNICKE, le syndrome de RETT, l'homocystéinémie, l'hyperprolinémie, l'hydroxybutirique-aminoacidurie, l'encéphalopathie de LEAD et la déficience en sulfite oxydase.

Les doses dépendent de l'effet recherché, de la durée du traitement et de la voie d'administration utilisée; elles sont généralement comprises entre 10 mg et 100 mg par jour par voie orale pour un adulte avec des doses unitaires allant de 5 mg à 50 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

## Revendications

1. Composés de formule : 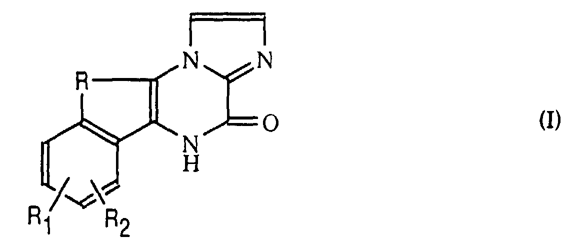 dans laquelle
- R représente un atome d'oxygène ou de soufre ou un radical NH ou N-alk,
- R₁ et R₂, identiques ou différents, représentent des atomes d'hydrogène ou d'halogène ou des radicaux alkyle, alcoxy, amino, acylamino, -NH-CO-NH-Ar, -N=CH-N(alk)alk', nitro, cyano, phényle, imidazolyle ou SO₃H,
- Ar représente un radical phényle,
- alk représente un radical alkyle,
- alk' représente un radical alkyle identique ou différent de alk,
étant entendu que les radicaux alkyle et alcoxy contiennent 1 à 4 atomes de carbone en chaîne droite ou ramifiée et les portions acyle contiennent 2 à 5 atomes de carbone,
les sels de ces composés et pour les composés pour lesquels R₁ et/ou R₂ représentent un radical -N=CH-N(alk)alk' leurs isomères (E et Z) ou les mélanges de ces isomères.

2. Composés de formule (I) selon la revendication 1 pour lesquels R₁ et R₂ représentent des atomes d'hydrogène et R représente un atome d'oxygène, de soufre ou un radical NH ou N-alk.

3. Procédé de préparation des composés de formule (I) selon la revendication 1 pour lesquels R représente un atome d'oxygène caractérisé en ce que l'on désalkyle, déshydrate et désalifie un dérivé de formule : 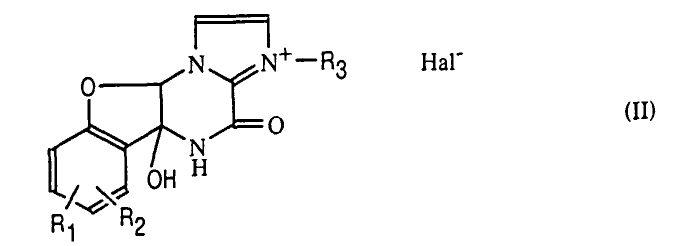 dans laquelle R₁ et R₂ ont les mêmes significations que dans la revendication 1, R₃ représente un radical alkyle et Hal représente un atome d'halogène, isole le produit et le transforme éventuellement en sel.

4. Procédé de préparation des composés de formule (I) selon la revendication 1 pour lesquels R représente un atome de soufre caractérisé en ce que l'on cyclise un dérivé de formule : 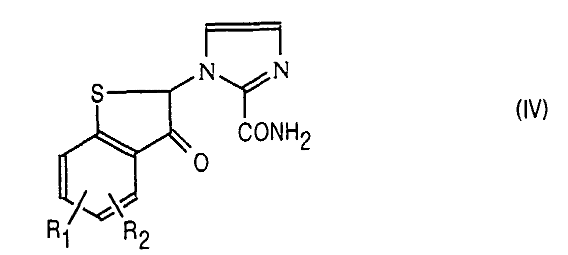 dans laquelle R₁ et R₂ ont les mêmes significations que dans la revendication 1, isole le produit et le transforme éventuellement en sel.

5. Procédé de préparation des composés de formule (I) selon la revendication 1 pour lesquels R représente un radical N-alk caractérisé en ce que l'on alkyle un composé de formule (I) correspondant pour lequel R représente un radical NH, isole le produit et le transforme éventuellement en sel.

6. Procédé de préparation des composés de formule (I) selon la revendication 1 pour lesquels R représente un radical NH caractérisé en ce que l'on hydrolyse un dérivé de formule : 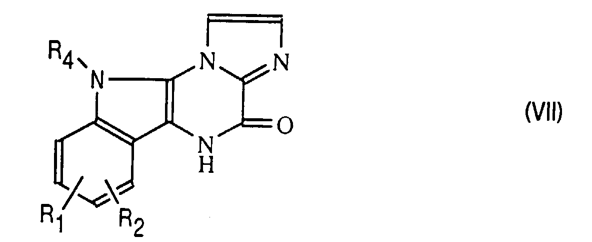 dans laquelle R₁ et R₂ ont les mêmes significations que dans la formule (I) et R₄ représente un radical acyle (2-5C), isole le produit et le transforme éventuellement en sel.

7. Médicaments contenant en tant que principe actif au moins un composé de formule (I) selon l'une des revendications 1 ou 2.

8. Médicaments selon la revendication 7 utiles comme antagonistes du récepteur AMPA.

9. Médicaments selon la revendication 7 utiles comme antagonistes du récepteur NMDA.

## Claims

1. Compounds of formula: 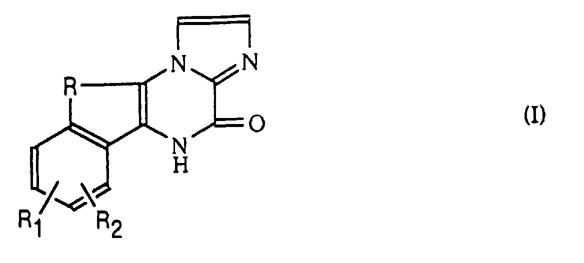 in which
- R represents an oxygen or sulphur atom or an NH or N-alk radical,
- R₁ and R₂, which are identical or different, represent hydrogen or halogen atoms or alkyl, alkoxy, amino, acylamino, -NH-CO-NH-Ar, -N=CH-N(alk)alk', nitro, cyano, phenyl, imidazolyl or SO₃H radicals,
- Ar represents a phenyl radical,
- alk represents an alkyl radical,
- alk' represents an alkyl radical which is identical to or different from alk,
it being understood that the alkyl and alkoxy radicals contain 1 to 4 carbon atoms in a straight or branched chain and the acyl portions contain 2 to 5 carbon atoms, the salts of these compounds and, for the compounds in which R₁ and/or R₂ represent an -N=CH-N(alk)alk' radical, their isomers (E and Z) or the mixtures of these isomers.

2. Compounds of formula (I) according to Claim 1 in which R₁ and R₂ represent hydrogen atoms and R represents an oxygen or sulphur atom or an NH or N-alk radical.

3. Process for the preparation of the compounds of formula (I) according to Claim 1 in which R represents an oxygen atom, characterized in that a derivative of formula: 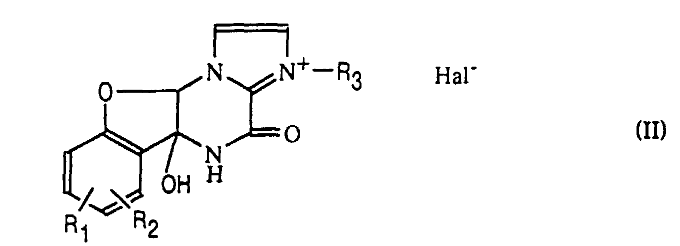 in which R₁ and R₂ have the same meanings as in Claim 1, R₃ represents an alkyl radical and Hal represents a halogen atom, is dealkylated, dehydrated and desalified, the product is isolated and optionally converted to a salt.

4. Process for the preparation of the compounds of formula (I) according to Claim 1 in which R represents a sulphur atom, characterized in that a derivative of formula: 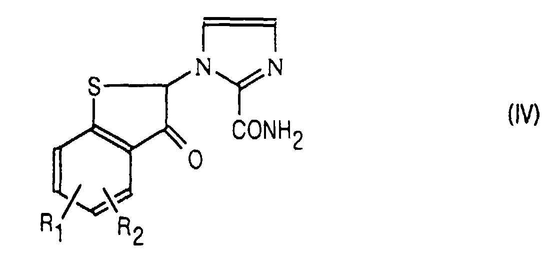 in which R₁ and R₂ have the same meanings as in Claim 1, is cyclized, the product is isolated and optionally converted to a salt.

5. Process for the preparation of the compounds of formula (I) according to Claim 1 in which R represents an N-alk radical, characterized in that a corresponding compound of formula (I) in which R represents an NH radical is alkylated, the product is isolated and optionally converted to a salt.

6. Process for the preparation of the compounds of formula (I) according to Claim 1 in which R represents an NH radical, characterized in that a derivative of formula: 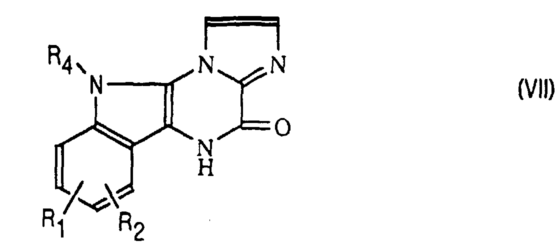 in which R₁ and R₂ have the same meanings as in the formula (I) and R₄ represents a (2-5C) acyl radical, is hydrolysed, the product is isolated and optionally converted to a salt.

7. Medicaments containing, as active principle, at least one compound of formula (I) according to either of Claims 1 and 2.

8. Medicaments according to Claim 7 which are useful as antagonists of the AMPA receptor.

9. Medicaments according to Claim 7 which are useful as antagonists of the NMDA receptor.

## Patentansprüche

1. Verbindungen der Formel (I) 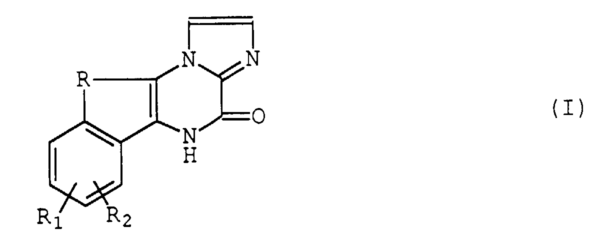 in der
- R ein Sauerstoffatom oder ein Schwefelatom oder einen Rest NH oder N-alk darstellt,
- R₁ und R₂, gleich oder verschieden, Wasserstoffatome oder Halogenatome oder Reste Alkyl, Alkoxy, Amino, Acylamino, -NH-CO-NH-Ar, -N=CH-N(alk)alk', Nitro, Cyano, Phenyl, Imidazolyl oder SO₃H bedeuten,
- Ar einen Phenylrest darstellt,
- alk einen Alkylrest darstellt,
- alk' einen mit alk identischen oder von alk verschiedenen Alkylrest bedeutet,
mit der Maßgabe, daß die Reste Alkyl und Alkoxy 1 bis 4 Kohlenstoffatome in gerader oder verzweigter Kette und die Acylteile 2 bis 5 Kohlenstoffatome enthalten,
die Salze dieser Verbindungen und bei den Verbindungen, worin R₁ und/oder R₂ einen Rest -N=CH-N(alk)alk' darstellen, ihre Isomere (E und Z) oder die Mischungen dieser Isomere.

2. Verbindungen der Formel (I) nach Anspruch 1, worin R₁ und R₂ Wasserstoffatome darstellen und R ein Sauerstoffatom, ein Schwefelatom oder einen Rest NH oder N-alk bedeutet.

3. Verfahren zur Herstellung der Verbindungen der Formel (I) nach Anspruch 1, worin R ein Sauerstoffatom darstellt, dadurch gekennzeichnet, daß man ein Derivat der Formel (II) 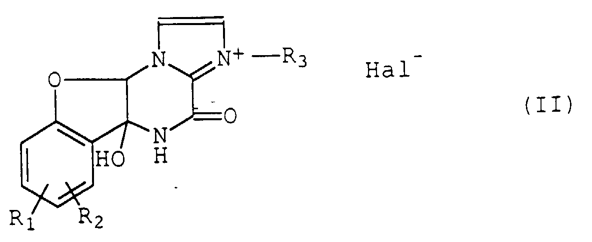 in der R₁ und R₂ die gleichen Bedeutungen wie in Anspruch 1 besitzen, R₃ ein Alkylrest ist und Hal ein Halogenatom bedeutet, dealkyliert, dehydratisiert und aus seinem Salz freisetzt, das Produkt isoliert und gegebenenfalls in Salz umwandelt.

4. Verfahren zur Herstellung der Verbindungen der Formel (I) nach Anspruch 1, worin R ein Schwefelatom darstellt, dadurch gekennzeichnet, daß man ein Derivat der Formel (IV) 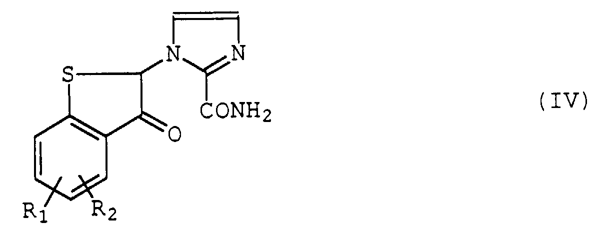 in der R₁ und R₂ die gleichen Bedeutungen wie in Anspruch 1 besitzen, cyclisiert, das Produkt isoliert und gegebenenfalls in Salz umwandelt.

5. Verfahren zur Herstellung der Verbindungen der Formel (I) nach Anspruch 1, worin R einen Rest N-alk darstellt, dadurch gekennzeichnet, daß man eine entsprechende Verbindung der Formel (I), in der R einen Rest NH bedeutet, alkyliert, das Produkt isoliert und gegebenenfalls in Salz umwandelt.

6. Verfahren zur Herstellung der Verbindungen der Formel (I) nach Anspruch 1, worin R einen Rest NH darstellt, dadurch gekennzeichnet, daß man ein Derivat der Formel (VII) 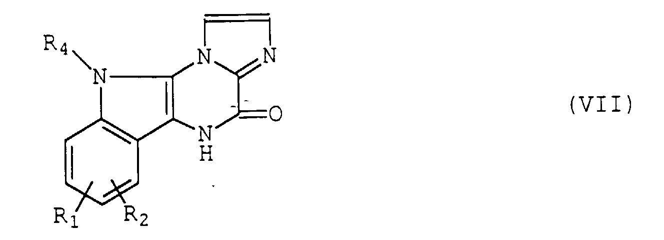 in der R₁ und R₂ die gleichen Bedeutungen wie in Anspruch 1 besitzen und R₄ einen Acylrest (2 bis 5 Kohlenstoffatome) darstellt, hydrolysiert, das Produkt isoliert und gegebenenfalls in Salz umwandelt.

7. Arzneimittel, die als Wirkstoff mindestens eine Verbindung der Formel (I) nach einem der Ansprüche 1 oder 2 enthalten.

8. Arzneimittel nach Anspruch 7, nützlich als Antagonisten des Rezeptors AMPA.

9. Arzneimittel nach Anspruch 7, nützlich als Antagonisten des Rezeptors NMDA.
